Europäisches Patentamt

European Patent Office

Office européen des brevets

(19)

(11) Publication number: **0 209 281**
**A1**

(12) **EUROPEAN PATENT APPLICATION**

(21) Application number: 86305029.0

(22) Date of filing: 27.06.86

(51) Int. Cl.⁴: **C 12 N 15/00,** C 12 P 21/02,
A 61 K 39/08

(30) Priority: 28.06.85 GB 8516442

(43) Date of publication of application: 21.01.87
Bulletin 87/4

(84) Designated Contracting States: **AT BE CH DE FR GB IT LI
LU NL SE**

(71) Applicant: **THE WELLCOME FOUNDATION LIMITED,
183-193 Euston Road, London NW1 2BP (GB)**

(72) Inventor: **Fairweather, Neil Fraser Wellcome Foundation
Ltd., Langley Court, Beckenham Kent (GB)**

(74) Representative: **Stott, Michael John et al, The Wellcome
Foundation Limited Group Patents & Agreements
Langley Court, Beckenham Kent BR3 3BS (GB)**

(54) **Cloned antigen.**

(57) The present invention relates to the cloning and expression of the gene encoding the <u>Clostridium tetani</u> neurotoxin (tetanus toxin) and to the use of the expressed protein in vaccines.

EP 0 209 281 A1

A750
0209281

## Cloned Antigen

The present invention relates to the cloning of the gene encoding the neurotoxin of Clostridium tetani, its expression and the use of the expression product in the manufacture of vaccines.

Tetanus is a highly infectious, world-wide disease caused by the organism Clostridium tetani. Its spores can remain dormant for indefinite periods in house dust and soil which are the prime sources of infection for open wounds. Contaminated animal faeces are also responsible for the spread of the disease and may further contaminate previously uninfected locales.

Tetanus infection is always a risk for the wounded and is usually associated with great pain and may even lead to death. Thus, in the nineteenth century, when the causative agent was discovered to be a bacterium, great effort was devoted to the development of an effective vaccine. The first successful vaccination in human subjects was carried out in 1926, when the vaccine used comprised crude tetanus toxin treated to render it atoxic.

Tetanus toxin provided the target for the first vaccine as it had already been discovered that symptoms are not due to the bacterium infecting host cells but rather by a potent neurotoxin released upon lysis of the bacterial cells.

To obtain sufficient toxin requires large cell-cultures which tends to give rise to toxin being contaminated with cell debris. The resulting toxin is rendered atoxic (toxoided) by treatment, usually with formaldehyde, but may also be toxoided with glutaraldehyde. However, while rendering the toxin harmless, either treatment may also lead to conjugation of the toxin with cell contaminants, which in turn may lead to possible adverse clinical reactions.

In vivo, a single (parent) peptide is synthesised, according to the genetic template, which comprises both light and heavy chains. On lysis of the bacterial cell, an endogenous protease cleaves the peptide between a pre-existing disulphide bridge to give two peptide chains linked by a disulphide bridge.

Papain cleaves the heavy chain to give a B-fragment (@ 100,000 MW) consisting

HDL/LMJ/11th June 1986

A750
0209281

of the light chain and part of the heavy chain, and a C fragment (@50,000 MW), consisting of the remainder of the heavy chain. It has been shown that both fragments are capable of providing protection but may be associated with residual toxicity which would be due to contamination with uncleaved toxin (Helting et al., J.Biol. Chem. (1977) 252, 187-193).

By "MW" is meant the apparent relative molecular weight as determined by polyacrylamide gel electrophoresis in the presence of sodium dodecyl sulphate and standard molecular weight markers. Molecular weight may thus be conveniently determined by the techniques described by U.K. Laemmli, Nature, (1970) 227, 680-685. Convenient standard molecular weight markers include, for example, phosphorylase B ($9.3 \times 10^4$ MW), bovine serum albumin ($6.8 \times 10^4$ MW), aldolase ($3.9 \times 10^4$ MW), triose phosphate isomerase ($2.7 \times 10^4$ MW), ovalbumin ($4.5 \times 10^4$ MW) and carbonic anhydrase ($2.9 \times 10^4$ MW).

It has now been discovered that the DNA sequence encoding tetanus toxin may be cloned and used to express tetanus toxin or an immunogenic fragment thereof. Thus, both B and C fragments, heavy and light chains and even the whole toxin may be obtained in greater yields and/or without one or more of the disadvantages of the prior art.

Thus, the present invention provides a cloned DNA sequence substantially encoding tetanus toxin or a peptide comprising at least one epitope thereof.

The term 'cloned' is used herein in reference to any DNA sequence which has been substantially synthesised outside of its natural host, either naturally or chemically.

The term 'peptide' as used herein pertains to any molecular structure composed primarily of amino acids and comprising more than 2 amino acids. It will be appreciated that tetanus toxin is a peptide by this definition.

The term 'epitope' as used herein denotes an immunogenic determinant of an immunogenic molecule, the immunogenic determinant comprising a molecular configuration capable of eliciting a protective immune response in a susceptible animal, when presented in a suitable form.

The DNA sequence according to the invention may correspond to any naturally occurring sequence encoding tetanus toxin, or it may correspond to a mutated

HDL/LMJ/11th June 1986

form of such a sequence, possible mutations including single or multiple base substitutions, deletions, insertions and inversions, the sequence, in any event, always encoding, in either positive or negative sense, at least one epitope of tetanus toxin.

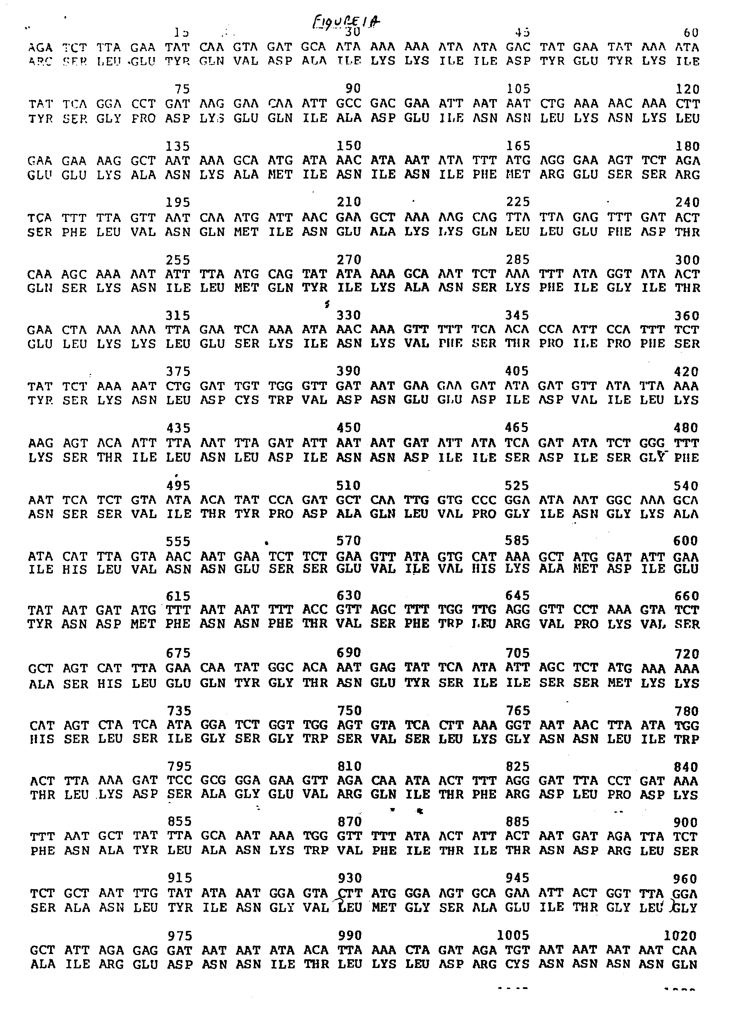

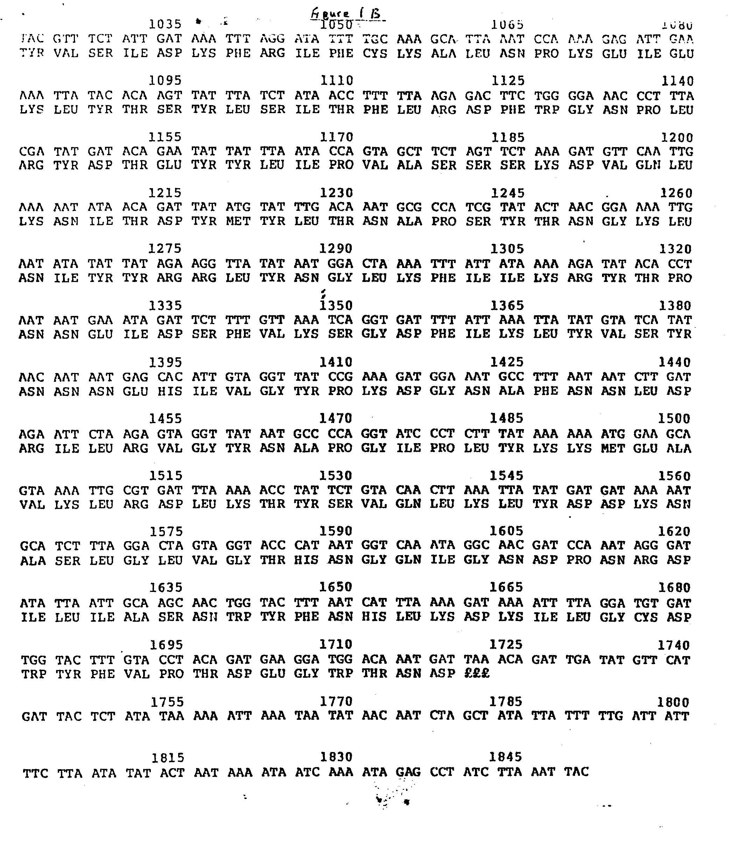

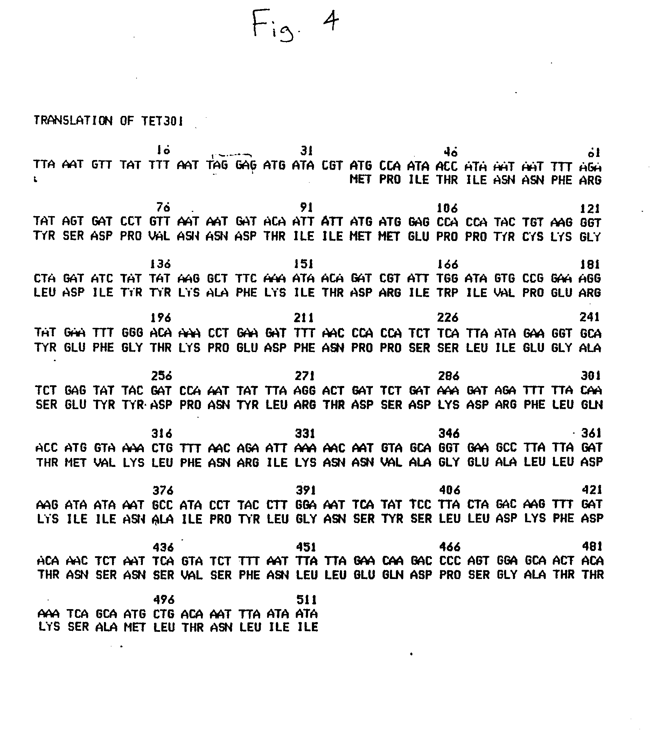

In a preferred aspect, the DNA sequence corresponds to at least part of that shown in Figure 1 or Figure 4, or is a sequence of substantial homology (at least 50%), the negative strand of which is capable of cross-hybridisation with that shown in Figure 1 or Figure 4, or is a sequence which differs from either of the fore-going only because of the degeneracy of the genetic code.

Moreover the DNA sequence according to the invention may encode a peptide of greater or lesser length than any desired peptide fragment as DNA manipulation techniques do not necessarily conveniently give rise to DNA sequences corresponding to desired peptides. Thus, for example, a DNA sequence substantially encoding the C fragment may not encode the first 30 amino acids of the fragment or, alternatively, it may also encode the last 30 amino acids of the B fragment.

A DNA sequence according to the invention may be obtained by the following procedure. A culture of C.tetani is grown for 3 days and then lysed in concentrated salt solution and tetanus toxin fragment C isolated and crystallised from the lysate. The oligopeptide N-terminal sequence of fragment C is then determined in an automated sequencer and a family of labelled oligonucleotides synthesised which correspond to possible DNA coding sequence for part of the oligopeptide. These oligonucleotides may then be used as a probe to identify a fragment of total-digest genomic DNA on nitrocellulose. Genomic DNA fragments of approximately the MW detected by the probe are then cloned into a suitable plasmid (e.g. pAT153) and used to transform a suitable host (e.g. E.coli JM101). Colonies are then picked and tested for hybridisation to the oligonucleotide probe. Any recombinant clone thus selected may then be used to generate further clones by using the insert DNA as a probe to identify further genomic DNA fragments. In this way, it is possible to generate a selection of clones encoding the entire tetanus toxin sequence. Excision of the inserts, treatment with nucleases and insertion into expression plasmids allows the expression of the fragments as peptides.

In a further embodiment of the invention, there is provided a synthetic peptide comprising at least one epitope of tetanus toxin.

HDL/LMJ/11th June 1986

In a yet further aspect of the invention there is provided a non-pathogenic virus provided with a DNA sequence according to the invention which may be used to provide immunity to tetanus in a susceptible vertebrate host by expressing the said sequence _in vivo_.

The invention also provides a non-pathogenic virus as defined above, further capable of providing immunity to other infection(s) by similar means, which may be administered jointly, or individually, together with any other vaccine.

In another feature of the present invention there is provided an expression vector, containing a DNA sequence according to the invention, tandemly linked to an amino-terminal coding portion of a gene translatable by the relevant host and, optionally, further comprising any control sequence(s) associated therewith.

In a further feature of the present invention there is provided a fusion peptide comprising the amino terminal portion of a host peptide, and the carboxy terminal being provided by at least one epitope of tetanus toxin.

In an alternative aspect of the invention, there is provided an expression vector comprising a gene capable of translation in a suitable host, optionally provided with relevant control sequences, into which is inserted a DNA sequence according to the invention suitably altered such that the portion of said gene carboxy-terminal to said DNA sequence is correctly translated on expression in a suitable host to produce a fusion protein comprising at least a part of tetanus toxin or a peptide comprising at least one epitope thereof and part of said gene-encoded protein.

According to a further feature of the present invention there is provided a method which comprises transforming a host cell with a cloning vector comprising the DNA sequence according to the invention and culturing the host cell to provide expression of tetanus toxin, or a peptide comprising at least one epitope thereof.

According to a further feature of the present invention there is provided a vector containing the DNA sequence according to the present invention.
In a further aspect of the invention, there is provided a vector containing the DNA sequence according to the invention, further containing one or more control sequences to regulate the expression of said DNA sequence.

HDL/LMJ/11th June 1986

In a further embodiment of the invention there is provided a method for synthesising at least a portion of tetanus toxin or a peptide comprising at least one epitope thereof, said tetanus toxin or peptide being optionally covalently linked to further peptide sequence, which method comprises the steps of:

a) creating a cDNA or genomic DNA library from Clostridium tetani;

b) selecting a probe for tetanus toxin DNA and rendering the said probe radio-active;

c) selecting a member or members of the said library by use of the said probe; and

d) using DNA thus selected from the said library to transform a suitable host which may be used to express the said tetanus toxin or a peptide comprising at least one epitope thereof.

In a further aspect there is provided a vaccine capable of inducing immunity to tetanus which comprises tetanus toxin, or a peptide comprising at least one epitope thereof, obtained by any process described herein, in association with a pharmaceutically acceptable carrier.

In a preferred aspect of the invention, there is provided a vaccine as described above, which comprises the B fragment, the C fragment, the light chain, the heavy chain and the toxin, or peptides substantially corresponding thereto, either singly or in any combination, when obtained by any process as described herein.

There is, in a further aspect, provided a method for inducing immunity to tetanus in a susceptible vertebrate host, comprising the administration of an effective amount of a vaccine, as hereinbefore defined, to the host.

The invention will be described in more detail hereinafter with reference to the accompanying drawings in which:

Figure 1 shows the base sequence of a stretch of C. tetani DNA and the corresponding amino acid sequence.

Figure 2 shows a restriction endonuclease map of part of the tetanus toxin gene and the relative position of certain clones.

Figure 3 is a less-detailed restriction map covering the entire tetanus toxin gene.

HDL/LMJ/11th June 1986

Figure 4 shows the base sequence of the DNA fragment encoding the N-terminus of the light chain of tetanus toxin.

Figure 1 (1A, 1B, 1C and 1D) shows the base sequence of a stretch of C. tetani DNA encoding part of the B fragment and all of the C fragment of tetanus toxin and the corresponding amino sequence. The 3 letter amino acid codes and one letter nucleotide codes are according to convention. The sequence was determined by methods described herein and it will be appreciated that, while it has been determined as accurately as possible within the margins of experimental error, there may be some variation between the sequence shown in the Figure and the actual tetanus toxin genetic sequence. The papain cleavage site is located at position 366.

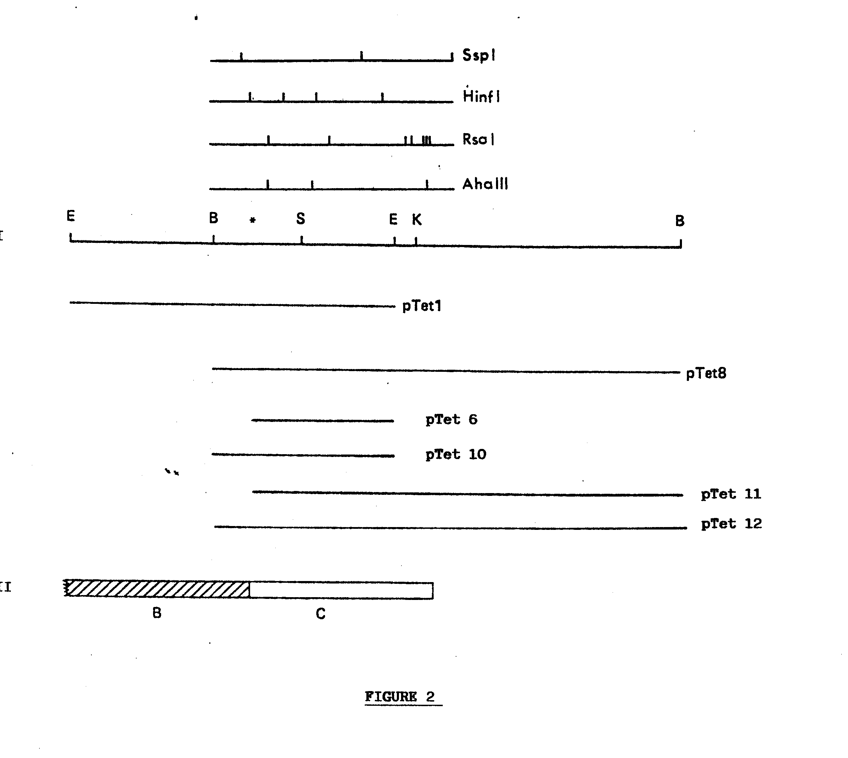

Figure 2 shows a restriction endonuclease map of the C fragment and part of the B fragment of tetanus toxin. The one-letter codes indicate restriction enzymes as follows: E (EcoRI); B (BglII); S (SacII) and K (KpnI). Other enzymes are as shown. * is used to indicate the site of the labelled oligonucleotide binding. Also shown is the relative size and position of the inserts of the indicated plasmids. The numeral I represents a stretch of C.tetani DNA, II represents the relevant position of part of the toxin and B and C indicate the position of the B and C fragments in the toxin.

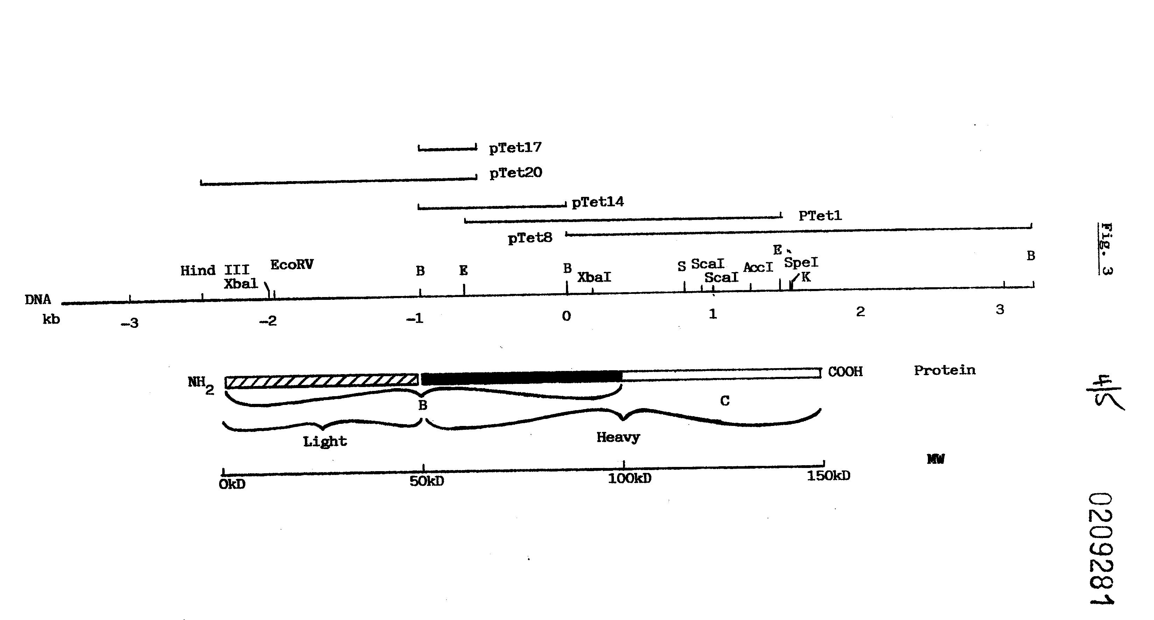

Figure 3 is an extended version of Figure 2 covering the whole gene. This figure is to scale and the scale of Figure 2 may be judged accordingly. Letter codes are as for Figure 2 but fewer restriction sites are noted due to the larger scale.

Figure 4 shows DNA and corresponding amino acid sequence for the N-terminus of the tetanus toxin light chain. The general description for Figure 1 also applies to Figure 4 insofar as codes and sequencing are concerned.

The DNA sequence according to the invention may also be characterised as having substantially the restriction map as shown in Figure 2 determined as described herein.

The genetic sequence was determined by the dideoxy method (Sanger, et al., (1977) Proc. Nat. Acad. Sci., 74, 5463-5467) after sub-cloning fragments of the target DNA into the bacteriophage cloning vectors M13mp8 (Messing, J. and Vieira, J; (1982) Gene 19, 269-276), M13mp18 and M13mp19 (Norrander, J., et al., Gene, 26, 101-106).

HDL/LMJ/11th June 1986

Using the sequence data shown in Figure 1, it will be appreciated that a peptide corresponding to any part of the sequence may be synthesised using for example, the methods described by Merrifield, R.B., and Marglin, A. (Ann. Rev. Biochem., 39, 841 et seq. (1970)).

The term 'synthetic' as used herein relates to peptides produced by a chemical method, for example as described above.

Cloning of the whole tetanus toxin gene may be carried out by the use of total genomic DNA digest 'libraries' which are prepared readily, and the relevant sequence, or fragments thereof, detected in such a library by the use of a suitable probe, e.g. see Example 2. A proportion of sequences found by this technique may be full length DNA sequences as required, though many will be fragments of such a sequence. In order to determine which clones in the library represent a part of the DNA sequence desired, a method known as 'chromosome walking' (Hadfield, C., Focus 5, 1-5 (1983) Bethesda Res. Labs.) may be employed, which method entails the use of known fragments (probes) to detect other fragments by cross-hybridisation. These freshly located sequences may themselves be used as probes and, in this way, the whole sequence of DNA substantially encoding tetanus toxin can be identified and cloned. Using such procedures, a restriction map characteristic of the DNA sequence may also be prepared.

The construction of a physical map of the gene for tetanus toxin in genomic DNA by restriction endonuclease cleavage, gel electrophoresis, transfer to nitrocellulose or polyamide membranes and hybridisation to specific probes derived from the cloned DNA is extremely useful to confirm the orientation and position of genomic clones and preparation of new clones.

Viruses may be used as hosts for the DNA sequence according to the invention. For example, a strain of vaccinia virus (Tk⁻) unable to confer upon infected cells the ability to grow on media not containing hypoxanthine is used to infect a tissue culture. The tissue culture may then be transformed with the tetanus toxin gene, or fragment(s) thereof, linked to a Tk$^+$ genetic determinant. Some of the subsequent viral progeny will have such transforming sequences in the form of inserts in their genomes. These can then be selected by their ability to

HDL/LMJ/11th June 1986

confer upon tissue culture cells the ability to grow on media devoid of hypoxanthine. Such colonies as do grow are then further selected for production of tetanus toxin, or peptides comprising at least one epitope thereof, for example by use of human immune serum. Such vaccinia strains can then be used to infect animals susceptible to tetanus, as the new vaccinia strain will cause production of a protective immunogenic tetanus peptide(s).

It will be appreciated that such vaccines are also readily capable of providing immunity to other infections, such as smallpox, diphtheria, hepatitis B, rabies, herpes simplex virus, whooping cough and the like, by incorporation of the relevant DNA sequences.

Based on the above-described characterisation of the DNA sequence for tetanus toxin the cloning of any desired fragment of the sequence is possible by reference to the DNA sequence or the restriction map so gained.

The insertion of a piece of foreign DNA into an E. coli gene in the correct reading frame allows the expression of a fusion protein, in which part of the amino acid sequence is derived from the E. coli gene and part of it is derived from the inserted DNA. Suitable expression vectors with appropriate control sequences and convenient restriction sites have been constructed, which allow high levels of expression of fusion proteins.

Thus, examination of the restriction map of the expression system of choice and the sequences to be expressed, together with a knowledge of the translational frame, enables specific DNA fragments to be ligated into the expression vector and expressed, without further manipulation. For example, pWRL507 is a plasmid constructed from pAT153 and the trpE gene (Nichols, B.P. et al., J. Mol. Biol. 146, 45-54 (1981)) with a synthetic EcoRI-Bgl II linker inserted at the Bgl II site at nucleotide 1223 in the trpE gene.

By using suitable restriction enzyme sites, DNA fragments from the tetanus toxin gene sequence may be cloned into a site within the trpE gene in the correct orientation, but usually in the wrong translational frame. In order to obtain expression of the inserted sequence, a synthetic linker of suitable length can be inserted into the restriction site between the trpE gene and the insert, to give in-frame expression of the fusion protein. Alternatively the plasmid

HDL/LMJ/11th June 1986

containing the inserted DNA can be opened at the unique restriction site between the bacterial and the inserted DNA and the DNA treated briefly with the enzyme Bal 31 to remove a few bases from each end of the linearised DNA. After repair with the large (Klenow) fragment of DNA polymerase I, the plasmid is recircularised with T4 ligase and used to transform bacteria. One in three of the transformants should contain the tetanus toxin sequence in the correct reading frame to be expressed as a fusion protein with the trpE gene product. It will be appreciated by those skilled in the art that the extent of digestion with Bal 31 will determine the final size of the expressed fusion protein and the relative lengths of trpE and tetanus toxin sequences contained therein. In addition, the insertion of a synthetic linker during ligation after Bal 31 digestion and repair facilitates the analysis of particular strains after transformation. By the judicious use of specific restriction enzyme digestion and Bal 31 enzyme treatment, any specific region of tetanus toxin can be expressed as a fusion protein.

Alternatively, nuclease S1, which preferentially degrades single-stranded DNA, can be used to digest away the 'sticky' end of the BglII restriction site to leave a 'blunt' end. Recircularisation of the plasmid will then put the inserted DNA in a new, and possibly correct, reading frame.

An alternative method for expressing DNA fragments is to use an open reading frame (ORF) vector into which (usually) short pieces of DNA can be inserted, often within the sequence coding for the N-terminal amino acid sequence of an E. coli protein. The inserted DNA must contain no stop codons in the correct translational frame, be in the correct orientation relative to the direction of transcription, and be in the correct frame at each end. For pieces of DNA from a protein coding sequence generated by a random cleavage method, the theoretical probability of read-through in the correct frame is 1 in 18. ORF vectors based on β-galactosidase have been described (Koenen et al., 1982). Insertion of a piece of DNA into a site at the N-terminus of the protein in the correct frame confers read-through expression of the β-galactosidase protein which can be detected by hydrolysis of the chromogenic substrate 5-bromo-4-chloro-3-indolyl-β-D-galactoside (Xgal). For example if Xgal is included in the agar upon which the colonies are grown, transformation of a suitable host strain with a plasmid expressing a functional β-galactosidase will produce a blue colony. One such vector, pXY460, contains the β-galactosidase gene under the

0209281

control of the tac promoter. Insertion of DNA into the Sma I site next to the EcoRI site may convert the gene to in-frame expression. Transformation of an E. coli host such as JM105 converts the bacteria to ampicillin resistance and expression of the fusion protein can be induced at high levels by the addition of isopropyl-β-D-thiogalactopyranoside (IPTG).

The peptide coded by part of the tetanus toxin gene in a fusion protein may be cleaved from that fusion protein by enzymic or chemical cleavage of the appropriate peptide bond. It will be apparent to those skilled in the art which enzymic or chemical cleavage method should be employed, by examination of the amino acid sequence expressed. By insertion of a synthetic oligonucleotide linker between the tetanus toxin DNA sequence and the bacterial gene sequence in the fusion protein expression system, a suitable site may be provided for enzymic or chemical cleavage between the tetanus toxin sequence and the remainder of the expressed fusion protein. In this way the tetanus toxin gene-encoded fragments may be purified away from host peptides.

Direct expression of the coding sequence for the tetanus toxin protein or parts thereof can be achieved by placing the inserted DNA sequence directly after an AUG start codon in the correct reading frame such that the DNA insert replaces the coding sequence normally transcribed and translated by the bacterial control region. Such a control region includes a promoter and a ribosome binding site in the optimal position relative to the start codon. The DNA sequence to be expressed may be correctly positioned by the use of suitable restriction sites and if necessary by using a suitable synthetic oligonucleotide linker. At the end of the inserted DNA sequence a stop codon may be inserted in the correct reading frame to stop translation and a terminator sequence to stop transcription may be added. The inserted DNA to be expressed may be the entire coding sequence of tetanus toxin or the entire sequence from which the amino-terminal signal sequence has been removed, or preferably a part of the coding sequence corresponding to an immunogenic fragment of the protein. Suitable fragments may be prepared by restriction enzyme digestion of a suitable DNA clone (after examination of the nucleotide sequence) and, if necessary, further treatment at either or both ends with Bal 31 to digest away parts of the DNA sequence in a controlled manner. Controlled digestion is achieved by the selection of proper buffer, temperature, reaction time and amount of enzyme, for example as described in Example 7. At this stage a suitable synthetic linker may be added, preferably by blunt end ligation to the insert, to provide an AUG start codon or facilitate ligation into the expression vector.

Controlled expression of any cloned fragment will be possible by use of the sequences at either end of the sequence, or by use of other sequences already known. Such sequences include promoters and enhancers. Examples of such promoters include lac, trp, bacteriophage λ pL and hybrid trp-lac (tac). Suitable enhancers include the SV40 enhancer and the enhancer from bovine papillomavirus.

The vector referred to above may be any appropriate vector which is suitable for the cloning of the DNA and which may be used to transform a host cell and thereby express the relevant protein. Such vectors include plasmids, bacteriophages and cosmids. Vectors which may be used for cloning cDNA include pUC8, pUC9, pAT153, pBR325 and pBR328 for use in Escherichia coli, pBD9 and pKT438 for use in Bacillus subtilis, pMA56 for use in yeast and pAdD26SV(A)-3, pSV2-dhfr, SVEHA3 and SVLHA8 for use in mammalian cells.

Vectors for use in expression of the relevant protein will include control sequences, such as mentioned above. Such vectors include pXY460 and pWRL 507 for use in E.coli or pSV2-dhfr for use in mammalian cells.

Examples of suitable host cells for use in the above-described method may be prokaryotic, such as bacteria (for example E.coli HB101 and DH1, B. subtilis sp.BD170 and IH6140), or eukaryotic, such as yeast (for example XV610-8C yeast cells) or mammalian cells (for example simian CV-1 cells).

The present invention further includes the said tetanus toxin protein or peptides comprising at least one epitope thereof, when obtained by any of the above methods according to the invention. These materials may be incorporated into a vaccine for conferring immunity against tetanus. For this purpose the antigenic protein, or a peptide comprising at least one epitope thereof, may be presented in association with a pharmaceutically acceptable carrier therefor. The antigenic proteins or peptides may be used either singly or in combination with other tetanus toxin epitope-containing peptides (e.g. C and B fragments together) or with other antigens which will provide immunity against tetanus.

HDL/LMJ/11th June 1986

Pharmaceutically acceptable carriers, in this instance, are liquid media suitable for use as vehicles to introduce the immune peptide into the patient. An example of such a carrier is saline solution. The tetanus toxin or peptide may be in solution or suspended as a solid in the carrier, or it may be solubilised by the addition of a pharmaceutically acceptable detergent(s).

The vaccine may also comprise an adjuvant for stimulating the immune response and thereby enhancing the effect of the vaccine. A convenient adjuvant for use in the present invention is aluminium hydroxide.

Conveniently the vaccines are formulated to contain a final concentration of tetanus toxin or peptide in the range of from 0.2 to 200 µg/ml, preferably 5 to 50 µg/ml, most preferably 15 µg/ml. After formulation the vaccine may be incorporated into a sterile container which is then sealed and stored at a low temperature, for example 4°C, or it may be freeze-dried.

In order to induce immunity in vertebrate hosts to tetanus one or more doses of the vaccine suitably formulated may be administered. It is recommended that each dose is 0.1 to 2 ml preferably 0.2 to 1 ml, most preferably 0.5 ml of vaccine.

The vaccines may be administered by any conventional method for the administration of vaccines including oral and parenteral (eg. subcutaneous or intramuscular) injection. The treatment may consist of a single dose of vaccine or a plurality of doses over a period of time.

The following examples are for illustration only and are not intended to limit the invention in any way.

Example 1: C. tetani DNA

C. tetani strain CN3911 was grown for 30 hours in 600 ml Mueller medium (Mueller, J.H., et al., J. Immunol. (1954) 50, 377-384). The cells were pelleted by centrifugation (10 min, 5000 rpm) using a Sorvall GS3 rotor and resuspended in 20 ml 50mM Tris-HC1 pH8.0, 5mM EDTA, 50mM NaCl (TES). The cells were centrifuged (10 min, 10,000rpm) in a Sorvall SS34 rotor and resuspended in 8 ml TES containing 25% sucrose. Lysozyme was added to 2 mg/ml and the cells incubated at 37°C for 20 min. 3.2ml 0.25M EDTA was added and incubation continued at 37°C for 25 min. The cells were lysed by the addition of 7.2 ml 2% Sarkosyl in TES followed by incubation for 10 min at 37°C and 10 min at 4°C. Protease K was added to 10 mg/ml and the lysate left overnight at 50°C. Eight ml of lysate was added to 35 ml of a solution containing 69.6 g CsCl and 55.2 ml 10mM Tris-HCl (pH8.0), 1mM EDTA (TE). PMSF was added to 50 µg/ml and the solution centrifuged at 36000 rpm for 48 hours at 20°C in a Beckman 70.1 Ti rotor. The C. tetani DNA, which was visible as opaque lumps in the clear solution, was withdrawn from the gradient using a wide-bore syringe and was dialysed extensively against TE. The DNA was extracted once with phenol and several times with ether followed by precipitation with ethanol at -20°C. The DNA was resuspended to 1 mg/ml and stored at -20°C.

Example 2: Crystalline Fragment C

Tetanus toxin was produced by lysing with 1M NaCl organisms obtained from a 3-day culture of the Harvard strain of C.tetani grown in Mueller medium. It was purified by fractional precipitation with potassium phosphate (1.75M, pH8.0) followed by adsorption to DEAE cellulose equilibrated with 0.01M sodium phosphate pH7.2, 0.2M EDTA. The purified fraction was equilibrated by dialysis against 0.1M Na phosphate pH6.5 0.1M EDTA. Fragment C was prepared from this material by the procedure of Helting and Zwisler (Helting, T.B., et al., J. Biol. Chem. (1977) 252, 187-193) using crystalline papain derived from crude enzyme according to the method of Kimmel and Smith (J. Biol. Chem. (1954) 207, 515-531).

Crystalline Fragment C was obtained by concentrating by vacuum dialysis either the appropriate fraction obtained from the Sephadex G100 column or the digestion mixture followed by dialysis against water. Several crops were obtained on further dialysis. Recrystallisation was achieved by dissolving the fragment C in 0.5M sodium chloride followed by dialysis against water. Almost quantitative yields of fragment C were obtained under these conditions. It was found that crystallisation reduced the residual toxicity of fragment C but did not eliminate it altogether even after twelve recrystallisations. It was always necessary to adsorb the fragment C with a column of adsorbed tetanus antitoxin coupled to Sepharose 4B as described by Helting and Zwisler (supra) in order to eliminate all residual toxicity. The purified fragment was finally dialysed as a crystalline suspension and freeze-dried.

HDL/LMJ/11th June 1986

## Example 3: Identification of Tetanus Toxin DNA

a)    Design of Synthetic Oligonucleotides

The sequence of the first 30 amino acids of purified fragment C was obtained by automated Edman degradation. The sequence obtained was: Lys,Asn,Leu,Asp,Cys,Trp,Val,Asp,Asn,Glu,Glu,Asp,Ile,Asp,Val,Ile,Leu,Lys, Lys,Ser,Thr,Ile,Leu,Asn,Leu,Asp,Ile,Asn,Asn,Asp. This was analysed for the longest possible stretch of amino acids giving the least degenerate oligonucletide mixture. The sequence of residues 6-11 and a family of 32 oligonucleotides, each of 17 bases length, is shown in Table 1 (where the bracketed nucleotides indicate possible variations). One of these oligonucleotides would be expected to be complementary to DNA and mRNA encoding fragment C. This family of oligonucletides was synthesised with mixtures of bases included where indicated and labelled with $^{32}$P-ATP.

### Table 1

| Amino Acid Sequence | Trp | Val | Asp | Asn | Glu | Glu | |
|---|---|---|---|---|---|---|---|
| Possible mRNA's | 5' UGG | GU(A) (G) (C) (U) | GA(U) (C) | AA(U) (C) | GA(A) (G) | GA | 3' |
| Oligonucleotides Synthesised | 3' ACC | CA(T) (C) | CT(A) (G) (G) (A) | TT(A) (G) | CT(T) (C) | CT | 5' |

b)       Identification and Cloning of C. tetani DNA Fragments

C. tetani chromosomal DNA was digested with several restriction enzymes, transferred to nitrocellulose and hybridised with the $^{32}$P-labelled oligonucleotide mixture. A prominent band of 2kb was identified in the EcoR1 digest and fainter higher molecular weight fragments with Pst1, Kpn1 and Hind III. 100 µg of C. tetani DNA was cleaved with EcoR1, electrophoresed on a

HDL/LMJ/11th June 1986

0.7% agarose gel and the DNA purified from the region containing fragments of approximately 2kb. This pool of 2kb EcoR1 fragments was cloned into EcoR1 cleaved and dephosphorylated plasmid pAT153.

One hundred recombinant clones were picked and the colonies were screened by hybridisation to the mixed oligonucleotide probe. Seven clones were identified showing varying degrees of reaction with the probe. Plasmid DNA was prepared from the seven clones and EcoR1 digests were probed by Southern blotting. Four plasmids contained 2.0kb EcoR1 inserts which hybridised to the oligonucleotide mixture and one of these plasmids, named pTet1, was studied further. A restriction map of thé insert in pTet1 is presented in figure 2. Further Southern blot experiments showed that only fragments containing the central 300 base pairs of the 2kb insert hybridised to the probe. Thus the sequence encoding the amino terminus of fragment C was located to the centre of the 2kb EcoR1 fragment.

DNA sequence analysis (See Example 3) revealed that the 2kb EcoR1 fragment did not contain the entire coding region of fragment C. Therefore, adjacent restriction enzyme-generated fragments were identified by southern blotting using pTet1 as a probe. A 3.2kb BglII C. tetani fragment was cloned into the vector pWRL507 at the BglII site to generate pTet8. This fragment contains the 1.4kb BglII-EcoR1 fragment present in pTet1 and the adjacent 1.8kb EcoR1-BglII fragment (see figure 2).

To obtain clones encoding the remainder of the B fragment of tetanus toxin (including the light chain), the C. tetani insert of pTet1 was used as a probe to yield a ≃ 1 kB Bgl II fragment of C. tetani DNA. This was cloned into pUC8 to form pTet14. pTet17 was then constructed by deleting the Eco R1 fragment from pTet14. The resulting 400 bp Eco R1-Hind III fragment from pTet17 was subsequently used to identify and clone, by similar procedures to the above, a 1.8 kbp C. tetani DNA fragment into pAt 153 to make pTet 20.

Sequencing of this fragment (Example 5, figure 4) shows a likely Shine and Dalgarno sequence (boxed) followed by open reading-frame from base 35. Thus, pTet 20 encodes the N-terminal of tetanus toxin and, together with pTet 1 and pTet 8, encodes the entire tetanus toxin gene.

Example 4: An Expression Plasmid Encoding the Entire C Fragment

Plasmids pTet6 and pTet10 do not encode the terminal 91 amino acids of fragment C. Derivatives of these plasmids which do contain the DNA encoding this region were constructed as follows. pTet8 was cleaved with restriction enzymes PstI and SacII and the 5.35 kb fragment purified. Similarly pTet6 and pTet10 were cleaved with PstI and SacII and fragments of 2.3kb and 2.75kb were purified. The PstI-SacII fragments of pTet8 and pTet6 were ligated and transformed into E. coli DH1 to form the recombinant plasmid pTet11 (7.7kb).

Similarly the PstI-SacII fragments of pTet8 and pTet10 were ligated to form pTet12 (8.1kb). pTet11 would be expected to express a fusion protein containing part of the trpE gene and 440 of the 451 amino acids of fragment C. pTet12 would be expected to express a fusion protein consisting of part of the trpE gene, 122 amino acids of fragment B and the whole of fragment C (451 amino acids). pTet11 and pTet12 produce cross-reacting proteins of 86,000MW and 101,000MW. These values are consistent with the structure of the plasmids and of the utilisation of the termination codon at nucleotide 1720 (figure 1). E.coli strain DH1 containing pTet12 has been deposited with the National Collection of Type Cultures, 61 Colindale Ave., London NW9 5HT on 28th June, 1985, and has been assigned the serial no. NCTC 11918.

Example 5: Nucleotide Sequence of the DNA Coding for Tetanus Toxin

Sequence analysis of the DNA was performed using the dideoxy procedure of Sanger (supra).

DNA templates were prepared by subcloning fragments of inserts in the filamentous phage cloning/sequencing vectors M13mp8 (Messing and Vieira, 1982, supra), M13mp18 or M13mp19 (Norrander, J., et al., Gene, (1983) 26, 101-106). The sequencing was performed using a synthetic universal primer (Celltech) and [$^{35}$S]-dATPαS (Amersham International) as described by Sanger et al. (supra). The basic strategy used to sequence specific fragments was to take specific restriction fragments (produced by digestion with any or a combination of RsaI, SauIIIA, SspI, EcoRI, BglII, SacII, AccI, AluI, KpnI, HinfI, Aha III) which were then purified by electro-elution, and where necessary, the staggered ends made blunt using the Klenow DNA polymerase I fragment. DNA

HDL/LMJ/11th June 1986

was ligated with phosphatase-treated endonuclease-digested cloning phage and transfected into E.coli JM101 (Messing, J., et al., Nucl. Acid Res. 9, 309 (1981)). Template DNA was prepared according to standard procedures. Wherever possible, sequence was obtained from both strands of each clone.

The sequence shown in figure 1 encodes the entire C fragment and the carboxy terminus of the B fragment. Translation of the entire sequence in both strands revealed only one open reading frame encoding a protein of 63kD. Translation of nucleotides 367-457 gives an amino acid sequence identical to that determined for the first 30 amino acids of fragment C, confirming that the clones do indeed encode a portion of tetanus toxin. The amino terminal residue of fragment C is lysine, which is in agreement with the results of Helting (Neubauer, V., Helting, T.B., Biochem. Biophys. Res. Com.(1977) 86, 635-642). The calculated molecular weight of fragment C is 51,562 daltons, a value in close agreement with our own measurements (data not shown) and with those of Helting (1977, supra).

Figure 4 shows the DNA sequence encoding the amino terminal portion of the light chain of tetanus toxin. The bases enclosed in a box correspond to the sequence (shine Dalgarno) required for ribosome-binding and the putative N-terminal amino acid (methionine) is encoded at base 35.

## Example 6: Restriction Map of Part of the Tetanus Toxin Gene

C. tetani DNA from plasmids pTet1 and pTet8 was prepared as described by Maniatis et al. ((1982) Molecular Cloning, A Laboratory Manual) and aliquots were restricted with specific endonucleases, either individually or on some occasions as a double digest, i.e. with two restriction endonucleases. The products were electrophoresed on agarose gels (0.5%) in Tris-Borate-EDTA (pH 8.2) containing 0.5 µg/ml Ethidium bromide and together with DNA fragments of known length as size markers in parallel tracks. An analysis of the size of restriction fragments from within the cloned DNA enabled a linear map of restriction enzyme sites to be constructed. A detailed map of the tetanus toxin gene corresponding to the C fragment is shown in Fig. 2, and a less-detailed map of the whole gene is shown in Fig. 3.

HDL/LMJ/11th June 1986

Example 7: TrpE-Tetanus Fusion Proteins.

Recombinant plasmid-containing strains of E. coli DH1 were grown overnight at 37°C in 10 ml M9 medium (Maniatis, T., et al. (1982) Molecular Cloning, A Laboratory Manual) containing 50 µg/ml ampicillin. The cultures were diluted 1 in 5 into fresh medium containing indoylacrylic acid (10 µg/ml) and grown for 4 hours with shaking. The cells were harvested by centrifugation and the polypeptides visualised by SDS polyacrylamide gel electrophoresis as described by Fairweather, N.F., et al. (Infection and Immunity (1982) 41, 1112-117). Western blotting was carried out as described (Towbin, H., et al., Proc. Nat. Acad. Sci. (1979) 76, 4350-4354) using 3% haemoglobin to block non-specific binding to nitrocellulose. Anti-fragment C125 antibody was used at a dilution of 1 in 50 and proteins were visualised using $^{125}$I protein A (50 000 cpm/ml).

The fusion proteins expressed would be expected to consist of amino terminal residues of the trpE product (anthranilate synthetase) and carboxyterminal residues of tetanus toxin. The 1.4kb BglII-EcoRl frgment of pTet1 was cloned into the BglII-EcoRl sites of pWRL507 to generate pTet4. No fusion protein containing tetanus toxin sequence was obtained as the BglII sites in pTet1 and pWRL507 are not in phase.

To generate fusion proteins in the same reading frame, two approaches were used.

1.      pTet4 was cut with BglII and digested with exonuclease Bal 31 for various times. After ligation and transformation into E. coli DH1, 100 colonies were picked and analysed by a solid phase immune screen for the presence of induced proteins reacting with anti-fragment C antibody. One clone containing a plasmid designated pTet6 was identified as reacting strongly with antibody (data not shown). The fusion protein was visualised by SDS polyacrylamide gel electrophoresis both as a stained band and by Western blotting. The size of the fusion protein, 70kD, is consistent with a deletion of approximately 400bp which was confirmed by restriction mapping (data not shown). DNA sequence analysis showed the exact size of the deletion to be 400 bp, the fusion protein encoded by nucleotides 163-1182 of trpE (Yanofsky, C.T., et al., Nuc. Acid Res.(1981) 9, 6647-6666) and nucleotides 399-1446 of tetanus DNA.

HDL/LMJ/11th June 1986

2.   The phase around the BglII site of pTet4 was also altered by using nuclease S1.  S1 preferentially degrades single stranded DNA and so should digest away the sticky ends of the BglII fragment to generate blunt ends.  Examination of the sequence around the BglII site of pTet4 shows that S1 treatment folowed by ligation should place the trpE and tetanus sequence in the same reading frame and should result in a trpE-tetanus fusion protein.  pTet4 was treated with BglII, S1 and T4 ligase and was transformed into DH1.  One transformant, containing a plasmid pTet10, which lacked a BglII site was examined for production of a hybrid protein upon induction.  A band of 93kD  was produced which reacted with anti-C fragment antibody.   The DNA in pTet4 does not encode the entire C fragment, lacking the 3' bases.  Derivatives of pTet6 and pTet10 were constructed which did contain the DNA encoding the entire C fragment. These plasmids, pTet11 and pTet12 also produced cross reacting fusion proteins of 86 and 101kD  respectively.  The sizes of the fusion proteins produced by these plasmids are consistent with the utilisation of the termination codon at nucleotide 1720 (see Figure 1).

3.   pTet13 was constructed from 2 plasmids, pXY460 and pTet8.  The 3.2kb BglII fragment, containing all the C fragment DNA and a piece of the B fragment DNA, was prepared from pTet8 and was ligated to pXY460 cleaved with BamHI.  The ligation mixture was introduced into E.coli TG1 by transformation, and strains containing recombinant plasmids were analysed by restriction enzyme digestion.  One plasmid, containing the 3.2kb BglII fragment inserted into pXY460 in the correct orientation and which expresses a protein of the predicted size (63kD) which reacts with anti-C fragment sera, was selected and named pTet13.

4.   pTet16 was constructed as follows.  The 3.2kb BglII fragment from pTet8 was purified and digested with SspI and Eco RI.  The resulting fragments were ligated to plasmids pUC9 cleaved with Eco RI.  The ligation reaction was introduced into E.coli TG1 by transformation, and pUC9 recombinants containing the Eco RI - SspI fragment containing the 3' end of the tetanus toxin gene were identified by restriction enzyme digestion.  One of these was selected and named pTet16.

5.   pTet18 was constructed by cleavage of pTet13 with KpnI and SalI and by ligation of this mixture of fragments to purified 320bp KpnI-SalI fragment from pTet16.  This ligation reaction was introduced by transformation

HDL/LMJ/11th June 1986

into E.coli. TG1 and recombinant plasmids were identified by restriction enzyme digestion and by reaction of the expressed proteins with anti-C fragment sera. One of the recombinant plasmids consisting of the KpnI-SalI fragment of pTet13 which contains most of the tetanus toxin C fragment DNA, and the 320bp KpnI-SalI fragment from pTet16 was selected and named pTet18.

## Example 8: Mouse Protection Experiments

Two recombinant plasmids were employed to test the ability of cloned DNA products, synthesised in E. coli, to protect mice against a lethal challenge of tetanus toxin.

1.  pTet11 (Example 7) produces a fusion protein consisting of E. coli trpE residues and tetanus toxin residues. All except the first 10 residues of fragment C are present on this protein.

2.  pTet18 (Example 7) expresses a portion of fragment B and all of fragment C as a mature protein. It is not fused to a long length of host protein.

a)  ### Protein Purification

The procedures for the purification of the proteins produced by pTet11 and pTet18 are different, as they are controlled by different promoters, trp and tac respectively.

1.  pTet11. An overnight culture of E.coli DH1 containing pTet11 was grown in 200 ml M9 medium. After overnight growth, a further 800 ml fresh medium was added together with 5 mg IAA (indoyl acrylic acid). Growth was continued for 4 hours.

2.  pTet18. An overnight culture of E.coli HB101 was grown in 100 ml L-broth. After overnight growth, a further 900 ml broth was added and the culture was grown for 5 hours.

The subsequent procedure for both purifications was as follows.

The cells were centrifuged (6000rpm, 10min) and the pellets stored overnight at 4°C.

HDL/LMJ/11th June 1986

The pellets were thawed at room temperature then placed on ice. After resuspension in a total volume of 16 ml of solution I (25 mM Tris-HC1 pH8.0, 1mM EDTA, 0.2% NP40, 1 mM PMSF), a further 4ml solution I containing 20 mg lysozyme was added and the mixture left on ice for 2 hours.

20µl 1M magnesium sulphate and 400µl 1mg/ml DNase were added and the mixture left a further 2 hours on ice. The cells were then pelleted at 13000 rpm, 10 min, 4°C and the supernatant (S1) stored at -20°C. The insoluble pellet was resuspended in 20ml 50mM Tris pH8.0, 5mM EDTA, 5mM EGTA, 1% NP40, 1mM PMSF and centrifuged as above. The supernatant (S2) was stored at -20°C. The pellet obtained after the second spin (designated FP2) was resuspended in 20ml 0.85% sodium chloride and dialysed overnight at 4°C.

The dialysed material (FP2) was frozen at -20°C.

Analysis by Western blotting revealed that the tetanus specific bands were enriched by this procedure.

b)      <u>Mouse Protection</u>

Mice were immunised with the FP2 protein prepared as follows.

<u>High dose</u>

10ml FP2 protein from trpE, pTet11 and pTet18 was centrifuged and the pellet resuspended in 1.6ml saline.

<u>Low dose</u>

2ml PF2 protein was centrifuged and resuspended in 1.6ml saline.

Tween 80 and Freund's complete adjuvant was added as described below.

1.6 ml pellet
0.4 ml 10% Tween 80
<u>2.0 ml</u> Freund's complete adjuvant
4.0 ml

HDL/LMJ/11th June 1986

0209281

Groups of 5 (Balb/c) mice were injected subcutaneously with 0.2 ml of protein/adjuvant mixture described above. Three weeks later a second injection was given. Five weeks after the second injection, the mice were challenged with approximately 100 $LD_{50}$ doses of tetanus toxin.

Control mice were injected with trpE protein, tetanus toxin fragment C or were not immunised at all. The results are shown in Table 2.

## Table 2

| Material | Amount per Injection(µg) | Survivors |
|---|---|---|
| pTet18 | High (125) | 5/5 |
| pTet18 | Low (25) | 5/5 |
| pTet11 | High (125) | 5/5 |
| pTet11 | Low (25) | 5/5 |
| TrpE | High (125) | 0/5 |
| TrpE | Low (25) | 0/5 |
| C fragment | ??? (5) | 5/5 |
| Not injected | - | 0/5 |

## Example 9: Formulations

a)    Intraperitoneal Combined Tetanus/Diphtheria/Whooping cough

Per 0.5 ml:
25 Lf Diphtheria toxoid
3.5 Lf Cloned Tetanus Toxin (10 µg protein)
$20 \times 10^9$ B. pertussis cells
20% alhydrogel
0.05 mg thiomersal
Sterile water q.s.

HDL/LMJ/11th June 1986

A750
0209281

b)   Intraperitoneal Diphtheria/Tetanus

This was prepared as above, with the omission of the pertussis component.

c)   Tetanus

per 0.5 ml:

7 Lf Cloned Tetanus Toxin (20 µg protein) 15% alhydrogel 0.05 mg thiomersal Sterile water q.s.

## Example 10: Toxicity Data

The insoluble fusion proteins were centrifuged and resuspended in 0.4ml 8M urea and injected subcutaneously.

2 mice were each given 1ml (250 µg protein), 2 mice were each given 5ml (1250 µg protein), 2 mice received 0.4ml 8M urea as a control.

No mice died.

The highest dose administered was at least 50 x higher than that required to induce effective immunity to tetanus.

HDL/LMJ/11th June 1986

Claims

1. A cloned DNA sequence substantially encoding tetanus toxin or a peptide comprising at least one epitope thereof.

2. A DNA sequence as claimed in claim 1 subsantially encoding the B or the C fragment of tetanus toxin, or at least one epitope thereof.

3. A DNA sequence as claimed in claim 1 substantially encoding the heavy or the light chain of tetanus toxin, or at least one epitope thereof.

4. A DNA sequence as claimed in claim 1 with at least 50% homology to either of Figures 1 and 4.

5. A vector containing a DNA sequence as claimed in any of claims 1 to 4.

6. An expression vector as claimed in claim 5, tandemly linked to an amino-terminal coding portion of a gene translatable by the vector's host cell.

7. An expression vector comprising a gene capable of translation in the vector's host cell containing a DNA sequence as claimed in any of claims 1 to 4, altered such that the portion of said gene carboxy-terminal to said DNA sequence is correctly translated on expression in the host to produce a protein comprising at least a part of tetanus toxin or a peptide comprising at least one epitope thereof fused to part of said gene-encoded protein.

8. A vector as claimed in any of claims 5 to 7 further comprising one or more control sequences.

9. A method for synthesising at least a portion of tetanus toxin or a peptide comprising at least one epitope thereof, said tetanus toxin or peptide being optionally convalently linked to further peptide sequence, which method comprises the steps of:

    a) creating a cDNA or genomic DNA library from Clostridium tetani;

b)     selecting a probe for tetanus toxin DNA and rendering the said probe radio-active;

c)     selecting a member or members of the said library by use of the said probe; and

d)     using DNA thus selected from the said library to transform a suitable host to express the said tetanus toxin or a peptide comprising at least one epitope thereof.

10.    A method for the synthesis of tetanus toxin or a peptide comprising at least one epitope thereof, comprising transforming a host cell with a cloning vector as claimed in any of claims 5 to 8 and culturing the host cell to provide expression of the said peptide.

11.    A synthetic peptide comprising at least one epitope of tetanus toxin.

12.    Tetanus toxin protein or a peptide comprising at least one epitope thereof, when obtained by a process according to either of claims 9 and 10.

13.    A fusion peptide comprising the amino terminal portion of a host peptide, and the carboxy terminal being provided by at least one epitope of tetanus toxin.

14.    A vaccine capable of inducing immunity to tetanus which comprises tetanus toxin, or a peptide comprising at least one epitope thereof, obtained by a process as claimed in either of claims 9 and 10, in association with a pharmaceutically acceptable carrier therefor.

15.    A vaccine as claimed in claim 14 comprising the B fragment, the C fragment, the light chain, the heavy chain or the toxin, or peptides substantially corresponding thereto, either singly or in any combination, when obtained by a process as claimed in either of claims 9 and 10.

HDL/LMJ/11th June 1986

A750CC
0209281

16. A method for inducing immunity to tetanus in a susceptible vertebrate host, comprising the administration of an effective amount of a vaccine, as claimed in either of claims 14 and 15, to the said host.

HDL/LMJ/11th June 1986

for Austria
für Österreich
pour l'Autriche

Claims

1. A method for synthesising at least a portion of tetanus toxin or a peptide comprising at least one epitope thereof, said tetanus toxin or peptide being optionally convalently linked to further peptide sequence, which method comprises the steps of:

   a)   creating a cDNA or genomic DNA library from <u>Clostridium tetani</u>;

   b)   selecting a probe for tetanus toxin DNA and rendering the said probe radio-active;

   c)   selecting a member or members of the said library by use of the said probe; and

   d)   using DNA thus selected from the said library to transform a suitable host to express the said tetanus toxin or a peptide comprising at least one epitope thereof.

2. A method as claimed in claim 1 wherein the transforming DNA substantially encodes tetanus toxin or a peptide comprising at least one epitope thereof.

3. A method as claimed in claim 1 wherein the transforming DNA subsantially encodes the B or the C fragment of tetanus toxin, or at least one epitope thereof.

4. A method as claimed in claim 1 wherein the transforming DNA substantially encodes the heavy or the light chain of tetanus toxin, or at least one epitope thereof.

5. A method as claimed in claim 1 wherein the transforming DNA has at least 50% homology to either of Figures 1 and 4.

6. A method as claimed in claim 1 wherein the transforming DNA comprises part of a vector.

HDL/LMJ/11th June 1986

7.  A method as claimed in claim 6, in which the transforming DNA is tandemly linked to an amino-terminal coding portion of a gene translatable by the vector's host cell.

8.  A method according to either of claims 6 and 7, wherein the vector further comprises a gene capable of translation in the vector's host cell, containing the said DNA sequence altered such that the portion of said gene carboxy-terminal to said DNA sequence is correctly translated on expression in the host to produce a protein comprising at least a part of tetanus toxin or a peptide comprising at least one epitope thereof fused to part of said gene-encoded protein.

9.  A method according to any of claims 6 to 8 in which the vector further comprises one or more control sequences.

10. Tetanus toxin protein or a peptide comprising at least one epitope thereof, when obtained by a method according to any of the preceding claims.

11. A fusion peptide comprising the amino terminal portion of a host peptide, and the carboxy terminal being provided by at least one epitope of tetanus toxin, when obtained by a method according to any of claims 1 to 9.

12. A method for inducing immunity to tetanus in a susceptible vertebrate host, comprising the administration of an effective amount of a vaccine containing tetanus toxin, or a peptide comprising at least one epitope thereof, obtained by a method according to any of claims 1 to 9, in association with a pharmaceutically acceptable carrier therefor.

13. A method according to claim 13 in which the vaccine comprises the B fragment, the C fragment, the light chain, the heavy chain or the toxin, or peptides substantially corresponding thereto, either singly or in any combination, when obtained by a method according to any of claims 1 to 9.

HDL/LMJ/11th June 1986

1/5

FIGURE 1A

```
           15                    30                    45                    60
AGA TCT TTA GAA TAT CAA GTA GAT GCA ATA AAA AAA ATA ATA GAC TAT GAA TAT AAA ATA
ARG SER LEU GLU TYR GLN VAL ASP ALA ILE LYS LYS ILE ILE ASP TYR GLU TYR LYS ILE

           75                    90                   105                   120
TAT TCA GGA CCT GAT AAG GAA CAA ATT GCC GAC GAA ATT AAT AAT CTG AAA AAC AAA CTT
TYR SER GLY PRO ASP LYS GLU GLN ILE ALA ASP GLU ILE ASN ASN LEU LYS ASN LYS LEU

          135                   150                   165                   180
GAA GAA AAG GCT AAT AAA GCA ATG ATA AAC ATA AAT ATA TTT ATG AGG GAA AGT TCT AGA
GLU GLU LYS ALA ASN LYS ALA MET ILE ASN ILE ASN ILE PHE MET ARG GLU SER SER ARG

          195                   210                   225                   240
TCA TTT TTA GTT AAT CAA ATG ATT AAC GAA GCT AAA AAG CAG TTA TTA GAG TTT GAT ACT
SER PHE LEU VAL ASN GLN MET ILE ASN GLU ALA LYS LYS GLN LEU LEU GLU PHE ASP THR

          255                   270                   285                   300
CAA AGC AAA AAT ATT TTA ATG CAG TAT ATA AAA GCA AAT TCT AAA TTT ATA GGT ATA ACT
GLN SER LYS ASN ILE LEU MET GLN TYR ILE LYS ALA ASN SER LYS PHE ILE GLY ILE THR

          315                   330                   345                   360
GAA CTA AAA AAA TTA GAA TCA AAA ATA AAC AAA GTT TTT TCA ACA CCA ATT CCA TTT TCT
GLU LEU LYS LYS LEU GLU SER LYS ILE ASN LYS VAL PHE SER THR PRO ILE PRO PHE SER

          375                   390                   405                   420
TAT TCT AAA AAT CTG GAT TGT TGG GTT GAT AAT GAA GAA GAT ATA GAT GTT ATA TTA AAA
TYR SER LYS ASN LEU ASP CYS TRP VAL ASP ASN GLU GLU ASP ILE ASP VAL ILE LEU LYS

          435                   450                   465                   480
AAG AGT ACA ATT TTA AAT TTA GAT ATT AAT AAT GAT ATT ATA TCA GAT ATA TCT GGG TTT
LYS SER THR ILE LEU ASN LEU ASP ILE ASN ASN ASP ILE ILE SER ASP ILE SER GLY PHE

          495                   510                   525                   540
AAT TCA TCT GTA ATA ACA TAT CCA GAT GCT CAA TTG GTG CCC GGA ATA AAT GGC AAA GCA
ASN SER SER VAL ILE THR TYR PRO ASP ALA GLN LEU VAL PRO GLY ILE ASN GLY LYS ALA

          555                   570                   585                   600
ATA CAT TTA GTA AAC AAT GAA TCT TCT GAA GTT ATA GTG CAT AAA GCT ATG GAT ATT GAA
ILE HIS LEU VAL ASN ASN GLU SER SER GLU VAL ILE VAL HIS LYS ALA MET ASP ILE GLU

          615                   630                   645                   660
TAT AAT GAT ATG TTT AAT AAT TTT ACC GTT AGC TTT TGG TTG AGG GTT CCT AAA GTA TCT
TYR ASN ASP MET PHE ASN ASN PHE THR VAL SER PHE TRP LEU ARG VAL PRO LYS VAL SER

          675                   690                   705                   720
GCT AGT CAT TTA GAA CAA TAT GGC ACA AAT GAG TAT TCA ATA ATT AGC TCT ATG AAA AAA
ALA SER HIS LEU GLU GLN TYR GLY THR ASN GLU TYR SER ILE ILE SER SER MET LYS LYS

          735                   750                   765                   780
CAT AGT CTA TCA ATA GGA TCT GGT TGG AGT GTA TCA CTT AAA GGT AAT AAC TTA ATA TGG
HIS SER LEU SER ILE GLY SER GLY TRP SER VAL SER LEU LYS GLY ASN ASN LEU ILE TRP

          795                   810                   825                   840
ACT TTA AAA GAT TCC GCG GGA GAA GTT AGA CAA ATA ACT TTT AGG GAT TTA CCT GAT AAA
THR LEU LYS ASP SER ALA GLY GLU VAL ARG GLN ILE THR PHE ARG ASP LEU PRO ASP LYS

          855                   870                   885                   900
TTT AAT GCT TAT TTA GCA AAT AAA TGG GTT TTT ATA ACT ATT ACT AAT GAT AGA TTA TCT
PHE ASN ALA TYR LEU ALA ASN LYS TRP VAL PHE ILE THR ILE THR ASN ASP ARG LEU SER

          915                   930                   945                   960
TCT GCT AAT TTG TAT ATA AAT GGA GTA CTT ATG GGA AGT GCA GAA ATT ACT GGT TTA GGA
SER ALA ASN LEU TYR ILE ASN GLY VAL LEU MET GLY SER ALA GLU ILE THR GLY LEU GLY

          975                   990                  1005                  1020
GCT ATT AGA GAG GAT AAT AAT ATA ACA TTA AAA CTA GAT AGA TGT AAT AAT AAT AAT CAA
ALA ILE ARG GLU ASP ASN ASN ILE THR LEU LYS LEU ASP ARG CYS ASN ASN ASN ASN GLN
```

Figure 1 B

TAC GTT TCT ATT GAT AAA TTT AGG ATA TTT TGC AAA GCA TTA AAT CCA AAA GAG ATT GAA
TYR VAL SER ILE ASP LYS PHE ARG ILE PHE CYS LYS ALA LEU ASN PRO LYS GLU ILE GLU

AAA TTA TAC ACA AGT TAT TTA TCT ATA ACC TTT TTA AGA GAC TTC TGG GGA AAC CCT TTA
LYS LEU TYR THR SER TYR LEU SER ILE THR PHE LEU ARG ASP PHE TRP GLY ASN PRO LEU

CGA TAT GAT ACA GAA TAT TAT TTA ATA CCA GTA GCT TCT AGT TCT AAA GAT GTT CAA TTG
ARG TYR ASP THR GLU TYR TYR LEU ILE PRO VAL ALA SER SER SER LYS ASP VAL GLN LEU

AAA AAT ATA ACA GAT TAT ATG TAT TTG ACA AAT GCG CCA TCG TAT ACT AAC GGA AAA TTG
LYS ASN ILE THR ASP TYR MET TYR LEU THR ASN ALA PRO SER TYR THR ASN GLY LYS LEU

AAT ATA TAT TAT AGA AGG TTA TAT AAT GGA CTA AAA TTT ATT ATA AAA AGA TAT ACA CCT
ASN ILE TYR TYR ARG ARG LEU TYR ASN GLY LEU LYS PHE ILE ILE LYS ARG TYR THR PRO

AAT AAT GAA ATA GAT TCT TTT GTT AAA TCA GGT GAT TTT ATT AAA TTA TAT GTA TCA TAT
ASN ASN GLU ILE ASP SER PHE VAL LYS SER GLY ASP PHE ILE LYS LEU TYR VAL SER TYR

AAC AAT AAT GAG CAC ATT GTA GGT TAT CCG AAA GAT GGA AAT GCC TTT AAT AAT CTT GAT
ASN ASN ASN GLU HIS ILE VAL GLY TYR PRO LYS ASP GLY ASN ALA PHE ASN ASN LEU ASP

AGA ATT CTA AGA GTA GGT TAT AAT GCC CCA GGT ATC CCT CTT TAT AAA AAA ATG GAA GCA
ARG ILE LEU ARG VAL GLY TYR ASN ALA PRO GLY ILE PRO LEU TYR LYS LYS MET GLU ALA

GTA AAA TTG CGT GAT TTA AAA ACC TAT TCT GTA CAA CTT AAA TTA TAT GAT GAT AAA AAT
VAL LYS LEU ARG ASP LEU LYS THR TYR SER VAL GLN LEU LYS LEU TYR ASP ASP LYS ASN

GCA TCT TTA GGA CTA GTA GGT ACC CAT AAT GGT CAA ATA GGC AAC GAT CCA AAT AGG GAT
ALA SER LEU GLY LEU VAL GLY THR HIS ASN GLY GLN ILE GLY ASN ASP PRO ASN ARG ASP

ATA TTA ATT GCA AGC AAC TGG TAC TTT AAT CAT TTA AAA GAT AAA ATT TTA GGA TGT GAT
ILE LEU ILE ALA SER ASN TRP TYR PHE ASN HIS LEU LYS ASP LYS ILE LEU GLY CYS ASP

TGG TAC TTT GTA CCT ACA GAT GAA GGA TGG ACA AAT GAT TAA ACA GAT TGA TAT GTT CAT
TRP TYR PHE VAL PRO THR ASP GLU GLY TRP THR ASN ASP ***

GAT TAC TCT ATA TAA AAA ATT AAA TAA TAT AAC AAT CTA GCT ATA TTA TTT TTG ATT ATT

TTC TTA ATA TAT ACT AAT AAA ATA ATC AAA ATA GAG CCT ATC TTA AAT TAC

FIGURE 2

Fig. 3

Fig. 4

TRANSLATION OF TET301

```
         16                    31                    46                    61
TTA AAT GTT TAT TTT AAT TAG GAG ATG ATA CGT ATG CCA ATA ACC ATA AAT AAT TTT AGA
                            MET PRO ILE THR ILE ASN ASN PHE ARG

         76                    91                   106                   121
TAT AGT GAT CCT GTT AAT AAT GAT ACA ATT ATT ATG ATG GAG CCA CCA TAC TGT AAG GGT
TYR SER ASP PRO VAL ASN ASN ASP THR ILE ILE MET MET GLU PRO PRO TYR CYS LYS GLY

        136                   151                   166                   181
CTA GAT ATC TAT TAT AAG GCT TTC AAA ATA ACA GAT CGT ATT TGG ATA GTG CCG GAA AGG
LEU ASP ILE TYR TYR LYS ALA PHE LYS ILE THR ASP ARG ILE TRP ILE VAL PRO GLU ARG

        196                   211                   226                   241
TAT GAA TTT GGG ACA AAA CCT GAA GAT TTT AAC CCA CCA TCT TCA TTA ATA GAA GGT GCA
TYR GLU PHE GLY THR LYS PRO GLU ASP PHE ASN PRO PRO SER SER LEU ILE GLU GLY ALA

        256                   271                   286                   301
TCT GAG TAT TAC GAT CCA AAT TAT TTA AGG ACT GAT TCT GAT AAA GAT AGA TTT TTA CAA
SER GLU TYR TYR ASP PRO ASN TYR LEU ARG THR ASP SER ASP LYS ASP ARG PHE LEU GLN

        316                   331                   346                    361
ACC ATG GTA AAA CTG TTT AAC AGA ATT AAA AAC AAT GTA GCA GGT GAA GCC TTA TTA GAT
THR MET VAL LYS LEU PHE ASN ARG ILE LYS ASN ASN VAL ALA GLY GLU ALA LEU LEU ASP

        376                   391                   406                   421
AAG ATA ATA AAT GCC ATA CCT TAC CTT GGA AAT TCA TAT TCC TTA CTA GAC AAG TTT GAT
LYS ILE ILE ASN ALA ILE PRO TYR LEU GLY ASN SER TYR SER LEU LEU ASP LYS PHE ASP

        436                   451                   466                   481
ACA AAC TCT AAT TCA GTA TCT TTT AAT TTA TTA GAA CAA GAC CCC AGT GGA GCA ACT ACA
THR ASN SER ASN SER VAL SER PHE ASN LEU LEU GLU GLN ASP PRO SER GLY ALA THR THR

        496                   511
AAA TCA GCA ATG CTG ACA AAT TTA ATA ATA
LYS SER ALA MET LEU THR ASN LEU ILE ILE
```

European Patent Office

**PARTIAL EUROPEAN SEARCH REPORT**
which under Rule 45 of the European Patent Convention shall be considered, for the purposes of subsequent proceedings, as the European search report

Application number

EP 86 30 5029

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | CHEMICAL ABSTRACTS, volume 104, no. 13, March 31, 1986, page 153 abstract 103262g COLUMBUS, OHIO (US) N.F. FAIRWEATHER et al.: "Cloning, nucleotide sequencing, and expression of tetanus toxin fragment C in Escherichia coli" & J. Bacteriol.1986,165(1),21-7 (Eng.) <br><br> * abstract * | 1-15 | C 12 N 15/00 <br> C 12 P 21/02 <br> A 61 K 39/08 |
| X | DE - A - 2 634 584 (BEHRINGWERKE AG) <br><br> * claims * | 14,15 | |
| Y | SCIENCE, volume 224, no. 4651 pages 881-884, WASHINGTON,DC (US) C.W. FINN et al.: "The structural gene for tetanus neurotoxin is on a plasmid" <br><br> * whole article * | 1 | **TECHNICAL FIELDS SEARCHED (Int. Cl.4)** <br><br> C 12 N <br> C 12 P <br> A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-15
Claims searched incompletely:
Claims not searched: 16
Reason for the limitation of the search:

Method for treatment of the human or animal body by surgery or therapy (see art. 52(4) of the European Patent Convention).

| Place of search | Date of completion of the search | Examiner |
|---|---|---|
| THE HAGUE | 24-09-1986 | GROSSKOPF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1505.1 03.82

| | **DOCUMENTS CONSIDERED TO BE RELEVANT** | | CLASSIFICATION OF THE APPLICATION (Int. Cl.4) |
|---|---|---|---|
| Category | Citation of document with indication, where appropriate, of relevant passages | Relevant to claim | |
| Y | BIOLOGICAL ABSTRACTS/RRM, Summary 28079500 PHILADELPHIA, Pa. (US) K. GORETZKI et al.: "Characterization of a plasmid encoding tetanus toxin" & Hoppe-Seyler's Zeitschrift für physiologische Cheme (DE) 1984, vol. 365, no. 9, page 992 <br><br> * whole abstract * | 1 | |
| A | CHEMICAL ABSTRACTS, volume 86, no. 10, March 7, 1977 page 176, abstract 67103e COLUMBUS, OHIO (US) T.B. HELTING et al.: "Structure of tetanus toxin.I. Breakdown of the toxin molecule and discrimination between polypeptide fragments" & J. Biol.Chem. 1977, 252(1) 187-93 (Eng) <br><br> * whole abstract * | 11,12 | TECHNICAL FIELDS SEARCHED (Int. Cl.4) |
| A | MICROBIOLOGICAL REVIEWS, volume 43, no. 2, pages 224-240 B. BIZZINI: "Tetanus toxin" <br><br> * page 227, last paragraph - page 229, last paragraph; table 2; page 234, right-hand column, second paragraph - page 240 * | 11,12 | |

EPO Form 1505.3   06.78